# EUROPEAN PATENT SPECIFICATION

(11) **EP 4 434 574 B1**
(45) Date of publication and mention of the grant of the patent: **05.08.2026**
(21) Application number: 24164946.6
(22) Date of filing: 20.03.2024
(51) Int. Cl.: A61N 1/372, A61N 1/375

(54) **BIOSTIMULATOR HAVING MOVABLE PACING ELEMENT**
BIOSTIMULATOR MIT BEWEGLICHEM SCHRITTMACHERELEMENT
BIOSTIMULATEUR AYANT UN ÉLÉMENT DE STIMULATION MOBILE

(30) Priority: 21.03.2023 US 202363453715 P; 19.03.2024 US 202418610143
(43) Date of publication of application: 25.09.2024
(73) Proprietor: Pacesetter, Inc., Sylmar, CA 91342 (US)
(72) Inventor: BORNZIN, Gene A, Sylmar, CA 91342 (US); SOMOGYI, Zoltan, Sylmar, CA 91342 (US)
(74) Representative: Barker Brettell Sweden AB

(56) References cited:
- WO-A1-2019/055850
- US-A1- 2014 324 145
- US-A1- 2017 106 185
- US-A1- 2020 289 835

## Description

### TECHNICAL FIELD

The present disclosure relates to biostimulators and related biostimulator transport systems. More specifically, the present disclosure relates to leadless biostimulators and related biostimulator transport systems useful for deep septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Deep septal pacing is an alternative to His-bundle pacing. Deep septal pacing involves pacing past the His-bundle toward the right ventricle apex. More particularly, deep septal pacing targets the left bundle branch below the His site. Deep septal pacing has been achieved using a lead in which the electrode penetrates several millimeters into the septum more than a centimeter below the His site. Remarkably, the deep septal pacing procedure is simple, pacing thresholds associated with deep septal pacing are potentially lower than with His-bundle pacing, and clinical efficacy of the approach has been demonstrated.

WO 2019/055850 discloses an implantable medical device having a housing and a fixation sheath including a housing sheath portion extending along an outer sidewall of the housing and a fixation member portion extending from the housing sheath portion. The housing sheath portion is advanceable from a retracted position, in which the fixation member portion is retracted, to a deployed position, in which the fixation member portion is deployed to extend away from a distal end of the housing for anchoring the implantable medical device at an implant site.

### SUMMARY

Existing leads are not well suited to pacing the deep septal area. Cadaver studies suggest that penetrating the membranous septum of the heart with such leads may not always be achieved. The leads lack the ability to control penetration into the target tissue, and thus, the leads may not penetrate beyond the fibrous endocardial tissue. Furthermore, the leads suffer from the shortcomings associated with conventional cardiac pacing systems. Accordingly, there is a need for a biostimulator and biostimulator system having a pacing element capable of accessing the deep septal area to deliver leadless, deep septal pacing to the heart.

The present invention is defined in the independent claim. Further embodiments of the invention are defined in the dependent claims.

A biostimulator is described. In an embodiment, the biostimulator includes a housing having a longitudinal axis and containing pacing circuitry in an electronics compartment. The biostimulator includes a fixation element coupled to the housing and an extendable support comprising a telescopic pedestal having a proximal pedestal end coupled to the housing and a distal pedestal end. The telescopic pedestal includes one or more stages telescopically movable relative to each other such that the distal pedestal end is longitudinally movable relative to the proximal pedestal end. The biostimulator includes a pacing element coupled to the distal pedestal end. The pacing element is longitudinally movable relative to the fixation element and the housing.

A biostimulator system is described. In an embodiment, the biostimulator includes a biostimulator transport system. The biostimulator system includes a biostimulator, as summarized above, mounted on the biostimulator transport system.

### BRIEF DESCRIPTION OF THE DRAWINGS

The novel features of the disclosure are set forth with particularity in the claims that follow. A better understanding of the features and advantages of the present disclosure will be obtained by reference to the following detailed description that sets forth illustrative embodiments, in which the principles of the disclosure are utilized, and the accompanying drawings.
FIG. 1 is a diagrammatic cross section of a patient heart illustrating an example implantation of a biostimulator in a target anatomy, in accordance with an embodiment.
FIG. 2 is a perspective view of a biostimulator system, in accordance with an embodiment.
FIG. 3 is a side view of a biostimulator having a movable pacing element, in accordance with an embodiment.
FIG. 4 is an end view of a biostimulator having a movable pacing element, in accordance with an embodiment.
FIG. 5 is a side view of a distal portion of a biostimulator having a pacing element movable relative to a fixation element, in accordance with an embodiment.
FIG. 6 is a cross-sectional view of a distal portion of a biostimulator having a pacing element movable relative to a fixation element, in accordance with an embodiment.
FIG. 7 is a cross-sectional view of an extendable support of a biostimulator in an unextended state, in accordance with an embodiment.
FIG. 8 is a cross-sectional view of an extendable support of a biostimulator in an extended state, in accordance with an embodiment.
FIG. 9 is a cross-sectional view, taken about line A-A of FIG. 7, of an extendable support of a biostimulator, in accordance with an embodiment.
FIG. 10 is a side view of a distal portion of a biostimulator having a pacing element movable relative to a fixation element, in accordance with an embodiment.
FIG. 11 is a cross-sectional view of an extendable support of a biostimulator in an unextended state, in accordance with an embodiment.
FIG. 12 is a cross-sectional view of an extendable support of a biostimulator in an extended state, in accordance with an embodiment.
FIG. 13 is a flowchart of a method of accessing a target anatomy using a biostimulator having a pacing element movable relative to a fixation element.

### DETAILED DESCRIPTION

Embodiments describe a biostimulator, such as a leadless pacemaker, having a pacing element that is movable relative to a fixation element. As described below, the biostimulator can be used to perform deep septal pacing of a heart. The biostimulator may, however, be used in other applications, such as deep brain stimulation. Thus, reference to the biostimulator as being a cardiac pacemaker for deep septal pacing is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator includes a pacing element that is longitudinally movable relative to a fixation element. More particularly, a relative longitudinal distance between the pacing element and the fixation element can change. For example, the pacing element can be mounted on an extendable support, such as a telescoping pedestal, which is capable of extending into a septal wall during device delivery. The biostimulator can be rotated when a helix of the pacing element is engaged in tissue to cause the telescoping pedestal to extend and advance into the septal wall. The pacing element may therefore advance to a target anatomy, such as a left bundle branch in the septal wall, by moving longitudinally relative to the fixation element. When the pacing element is extended into the target anatomy, the fixation element can be anchored in the septal wall, e.g., at a right bundle branch. Pacing of the bundle branch(es) may be provided by the pacing element and/or the fixation element.

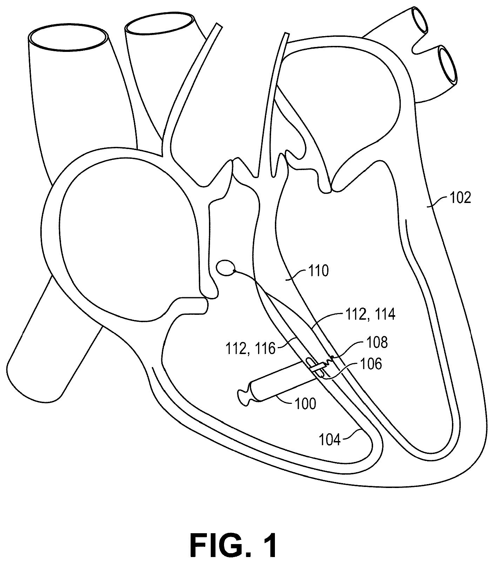

Referring to FIG. 1, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 100. The biostimulator 100 can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to a septum 104 of the heart 102. More particularly, the biostimulator 100 can be delivered to the septum 104, and one or more elements, such as a fixation element 106 and/or a pacing element 108 can pierce a septal wall 110 of the septum 104 to engage and anchor the biostimulator 100 to the target anatomy, e.g., a bundle branch 112 in the septal wall 110. In a particular embodiment, the biostimulator 100 can use two or more electrodes located on or within a housing of the biostimulator 100 for pacing the cardiac chamber upon receiving a triggering signal from at least one other device within the body. In an embodiment, one or more of the fixation element 106 or the pacing element 108 is an active electrode.

When the biostimulator 100 is delivered to and screwed into the septum 104 of the heart 102, the pacing element 108 and/or the fixation element 106 may be positioned for deep septal pacing at respective bundle branches 112 in the septum 104. For example, an active electrode of the pacing element 108 can be positioned at a first target anatomy in the septal wall 110, e.g., a left bundle branch 114. Similarly, the fixation element 106 can be positioned at a second target anatomy in the septal wall, e.g., a right bundle branch 116. Optionally, one of the elements may be at a bundle branch and the other element may not be at a bundle branch.

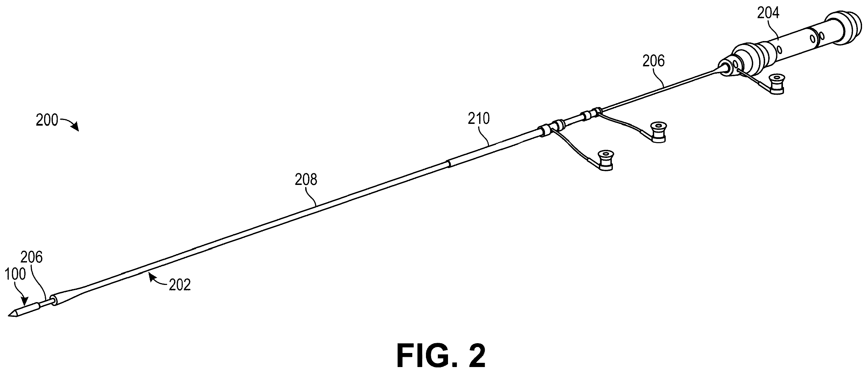

Referring to FIG. 2, a perspective view of a biostimulator system is shown in accordance with an embodiment. A biostimulator system 200 can include the biostimulator 100, e.g., a leadless pacemaker or other leadless biostimulator, and a biostimulator transport system 202. The biostimulator transport system 202 can be used to deliver or retrieve the biostimulator 100 from a patient. The biostimulator system 200 can include delivery or retrieval systems, which may be catheter-based systems used to carry the leadless biostimulator 100 intravenously to or from a patient anatomy. For example, the biostimulator 100 can be attached, connected to, or otherwise mounted on the biostimulator transport system 202. The biostimulator 100 can be mounted on a distal end of a catheter of the biostimulator transport system 202. The biostimulator 100 is thereby advanced intravenously into or out of the heart 102.

The biostimulator transport system 202 can include a handle 204 to control movement and operations of the transport system from outside of a patient anatomy. One or more elongated members extend distally from the handle 204. For example, a support member 206 can extend distally from the handle 204. The support member 206 can extend to a distal end of the transport system 202. In an embodiment, the biostimulator 100 is mounted on the biostimulator transport system 202, e.g., at a distal end of the support member 206.

The biostimulator transport system 202 can include a protective sleeve 208 to cover the biostimulator 100 during delivery and implantation. The protective sleeve 208 can extend over, and be longitudinally movable relative to, the support member 206. The biostimulator transport system 202 may also include an introducer sheath 210 that can extend over, and be longitudinally movable relative to, the protective sleeve 208. The introducer sheath 210 can cover a distal end of the protective sleeve 208, the support member 206, and the biostimulator 100 as those components are passed through an access device into the patient anatomy.

Several components of the biostimulator transport system 202 are described above by way of example. It will be appreciated, however, that the biostimulator transport system 202 may be configured to include additional or alternate components. More particularly, the biostimulator transport system 202 may be configured to deliver and/or retrieve the biostimulator 100 to or from the target anatomy. Delivery and/or retrieval of the biostimulator 100 can include retaining the biostimulator 100 during transport to the target anatomy and rotation of the biostimulator 100 during implantation of the biostimulator at the target anatomy. Accordingly, the biostimulator transport system 202 can incorporate features to retain and rotate the biostimulator 100.

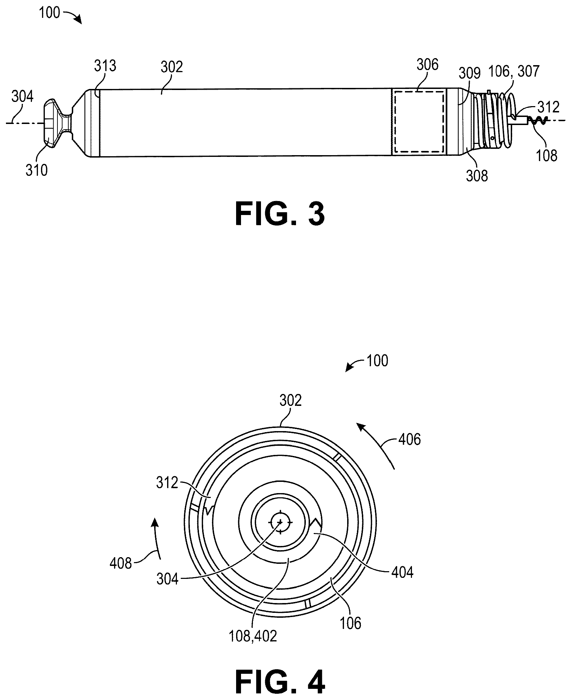

Referring to FIG. 3, a side view of a biostimulator having a movable pacing element is shown in accordance with an embodiment. The biostimulator 100 can be a leadless cardiac pacemaker that can perform cardiac pacing and that has many of the advantages of conventional cardiac pacemakers while extending performance, functionality, and operating characteristics. The biostimulator 100 can have two or more electrodes, e.g., a portion of the pacing element 108 that acts as an active electrode and/or a portion of the fixation element 106 that acts as an active electrode. The electrodes can deliver pacing pulses to bundle branch(es) 112 within the septal wall 110 of the heart 102 to perform deep septal pacing, and optionally, can sense electrical activity from the muscle. The electrodes may also communicate bidirectionally with at least one other device within or outside the body.

In an embodiment, the biostimulator 100 includes a housing 302 having a longitudinal axis 304. The housing 302 can contain a primary battery to provide power for pacing, sensing, and communication, which may include, for example, bidirectional communication. The housing 302 can optionally contain an electronics compartment 306 (shown by hidden lines) to hold pacing circuitry adapted for different functionality. For example, pacing circuitry in the electronics compartment 306 can include circuits for sensing cardiac activity from the electrodes, circuits for receiving information from at least one other device via the electrodes, circuits for generating pacing pulses for delivery to tissue via the electrodes, or other circuitry. The electronics compartment 306 may contain circuits for transmitting information to at least one other device via the electrodes and can optionally contain circuits for monitoring device health. The circuit of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

Leadless pacemakers or other leadless biostimulators 100 can be fixed to an intracardial implant site, e.g., at the septal wall 110, by one or more actively engaging mechanisms or fixation mechanisms, such as a screw or helical member that screws into the myocardium. In an embodiment, the biostimulator 100 includes the fixation element 106 coupled to the housing 302. The fixation element 106 can include a proximal helix 307 to screw into target tissue. More particularly, the biostimulator 100 can include a header assembly having a flange 308 coupled to a distal housing end 309 of the housing 302. The fixation element 106 can extend helically from the flange 308. More particularly, the proximal helix 307 can extend from the flange 308 about the longitudinal axis 304 to a first helix tip 312. Accordingly, when the biostimulator 100 is delivered to the target tissue, the first helix tip 312 can pierce the tissue and the housing 302 can be rotated to screw the fixation element 106 into the target tissue to place the proximal helix 307 in contact with the tissue.

In an embodiment, the biostimulator 100 includes an attachment feature 310. The attachment feature 310 can be mounted on a proximal housing end 313 of the housing 302. More particularly, the attachment feature 310 can be mounted on an opposite end of the housing 302 from the fixation element 106 and the pacing element 108, which can instead be coupled to the distal housing end 309 of the housing 302. The attachment feature 310 can facilitate precise delivery or retrieval of the biostimulator 100. For example, the attachment feature 310 can be formed from a rigid material to allow the transport system 202 to engage the attachment feature 310 and transmit torque to the attachment feature 310 to screw one or more of the fixation element 106 or the pacing element 108 into the target tissue.

Referring to FIG. 4, an end view of a biostimulator having a movable pacing element is shown in accordance with an embodiment. The biostimulator 100 can include the pacing element 108 coupled to the housing 302. The pacing element 108 can be coaxially arranged with the fixation element 106 about the longitudinal axis 304, and can be coupled to the distal housing end 309 of the housing 302. More particularly, the pacing element 108 can extend along, e.g., axially along or helically about, the longitudinal axis 304 at a location that is radially inward from the fixation element 106. Furthermore, the pacing element 108 may be located distal to the fixation element 106, and can include distal helix 402 extending about the longitudinal axis 304. More particularly, the distal helix 402 can extend about the longitudinal axis 304 to a second helix tip 404, which may be distal to the first helix tip 312.

The pacing element 108 and the fixation element 106 can include respective helical elements to screw into a target tissue, as described below. Accordingly, when the helical tip(s) of the helical element(s) are engaged to tissue and the housing 302 is rotated in a first rotational direction 406, e.g., clockwise, the element(s) 106, 108 can be driven through the septal wall 110 tissue to advance toward the bundle branch 112. By contrast, rotation of the housing 302 in a second rotational direction 408 opposite to the first rotational direction 406, e.g., counterclockwise, can cause the element(s) 106, 108 to retract from the bundle branch 112. Accordingly, rotation of the housing 302 can control advancement or retraction of the pacing element 108 and/or the fixation element 106 into the septal wall 110 of the heart 102.

One or more of the fixation element 106 or the pacing element 108 can be an active electrode, and can electrically communicate with the pacing circuitry contained in the electronics compartment 306. Accordingly, the anchored element(s) can electrically communicate with the tissue, and can transmit electrical pulses between the tissue and the circuitry of the biostimulator 100. To facilitate electrical function of the fixation element 106 and/or the pacing element 108, the element(s) may be coated in titanium nitride to reduce a polarization value of the electrode(s). By way of example, the titanium nitride coating may be in a range of 5 to 50 microns, e.g., 13 microns.

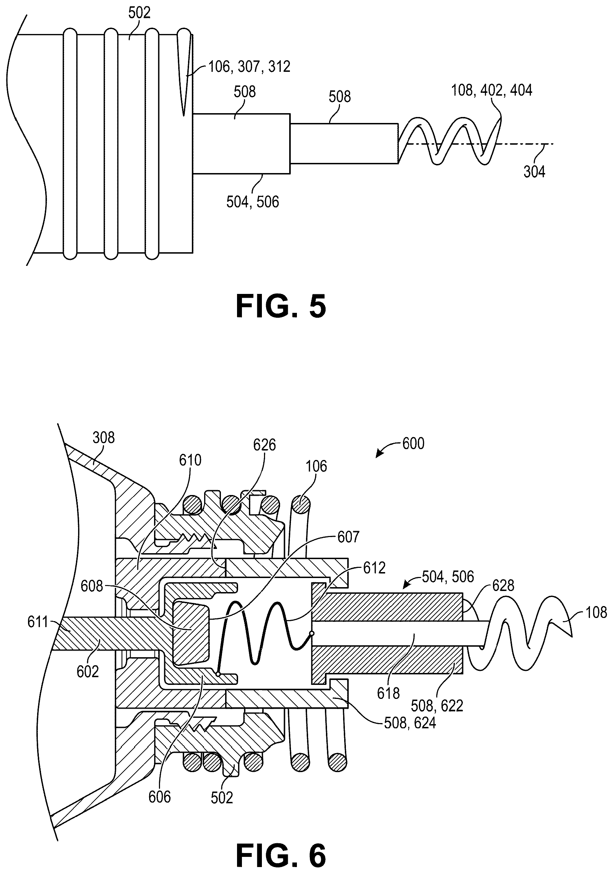

Referring to FIG. 5, a side view of a distal portion of a biostimulator having a pacing element movable relative to a fixation element is shown in accordance with an embodiment. The fixation element 106 can include the proximal helix 307 mounted on a non-conductive helix mount 502. The helix mount 502 can be formed from polyether ether ketone (PEEK). The fixation element 106 can include MP35N wire having a wire diameter of 0.4-0.6 mm, e.g., 0.5 mm. The wire may be formed from a metal, such as stainless steel or a nickel cobalt alloy. The wire can be wrapped into the helix having several turns extending to the first helix tip 312. The first helix tip 312 can penetrate the septal wall 110 and the turns can be screwed into the septum 104 to anchor the biostimulator 100 within the heart 102.

The pacing element 108 of the biostimulator 100 can include an elongated screw. More particularly, the pacing element 108 can include a helical electrode extending about the longitudinal axis 304 to the second helix tip 404. In an embodiment, the pacing element helix includes platinum-iridium wire (90-10 wire) having a diameter of 0.25-0.31 mm, e.g., 0.28 mm diameter. A major diameter of the helix can be 1.2-1.6 mm, e.g., 1.4 mm. A length of the distal helix 402 may be in a range of 5-20 mm, e.g., 7 mm, 12 mm, 15 mm, or 20 mm. The distal helix 402 can provide a means of pacing a target tissue that is distal to tissue being engaged by the fixation element 106. For example, the second helix tip 404 can penetrate the septal wall 110 and the turns of the distal helix 402 can be screwed into the septum 104 to pace the left bundle branch 114 when the fixation element 106 is engaged near a surface of the septal wall 110 and/or to the right bundle branch 116.

In an embodiment, the pacing element 108 is movable relative to the fixation element 106. For example, the pacing element 108 can be mounted on an extendable support 504 that can expand in a longitudinal direction, along the longitudinal axis 304, over an expansion range. More particularly, the extendable support 504 can lengthen or contract (shorten) such that a longitudinal distance between the pacing element 108 and the fixation element 106 varies. The extension range may be a distance between 5 to 14 mm, for example. Accordingly, when the extendable support 504 is in an unextended or collapsed state, the distance separating the elements can be 5 mm, and when the extendable support 504 is in an extended or expanded state, the distance separating the elements can be 14 mm. The pacing element 108 can therefore move longitudinally relative to the fixation element 106 to extend into the septal wall 110, away from the fixation element 106, to pace the left bundle branch 114.

The extendable support 504 may include a telescopic pedestal 506. The telescopic pedestal 506 of the biostimulator can have one or more stages 508 that slide or otherwise move relative to each other to elongate the extendable support 504. Accordingly, the telescopic pedestal 506 can provide a mechanism by which the pacing element 108 can move longitudinally relative to the fixation element 106. The telescopic pedestal 506 is described further below.

Referring to FIG. 6, a cross-sectional view of a distal portion of a biostimulator having a pacing element movable relative to a fixation element is shown in accordance with an embodiment. In an embodiment, the biostimulator includes a header assembly 600. The header assembly 600 can be mounted on the housing 302, e.g., at the distal housing end 309, and can include both the fixation element 106 and the pacing element 108. The fixation element 106 can be electrically connected to the housing 302, which may be insulated. Alternatively, the fixation element 106 can be electrically connected to pacing circuitry within the housing 302 through an electrical feedthrough 602 of the header assembly 600. More particularly, the electrical feedthrough 602 can be electrically connected to circuitry within the electronics compartment 306, and the fixation element 106 can be electrically connected to the electrical feedthrough 602. Accordingly, the circuitry can be controlled to polarize the fixation element 106 to provide either anodic or cathodic pacing of the target tissue.

In an embodiment, the header assembly 600 includes a helix mount 502, which can be mounted on the housing 302. For example, the helix mount 502 can have an internal thread that mounts on an external thread of the flange 308 by a threaded connection (not shown). The fixation element 106 may then be mounted on the helix mount 502, e.g., by screwing the helical structure onto external threads of the helix mount 502.

The electrical feedthrough 602 of the header assembly 600 can include an electrode cup 606 having an electrode wall extending distally from an electrode base. The electrode wall can extend around an electrode cavity 607 to surround a filler 608 and separate the filler 608 from an insulator 610 or the flange 308 that is radially between the electrode cup 606 of the feedthrough and the helix mount 502. The filler 608 can contain a therapeutic agent. The electrode cavity 607 can be located on the longitudinal axis 304. A pin 611 can extend proximally from the electrode cup 606 along the longitudinal axis 304 through the insulator 610. The pin 611 can receive pacing impulses from circuitry within the electronics compartment 306 and transmit the electrical signals to the electrode cup 606. The impulse may then transmit into the pacing element 108, e.g., through an extendable conductor 612. The extendable conductor 612 is described further below and can be electrically connected to the electrode cup 606 and the pacing element 108. More particularly, the extendable conductor 612 can electrically connect the pacing circuitry to the pacing element 108. The electrical connection may be made through the telescopic pedestal 506. For example, the telescopic pedestal 506 may include a conductive core 618 to convey the impulses from a distal end of the extendable conductor 612 to a proximal end of the pacing element 108. The pacing element 108 may then deliver the pacing impulses to the target tissue, e.g., the left bundle branch 114.

As described above, the pacing element 108 may be electrically connected to the pacing circuitry. Similarly, in an embodiment, the fixation element 106 may be electrically connected to the pacing circuitry. More particularly, the electrical feedthrough 602 can deliver an impulse to the fixation element 106. The fixation element 106 may be electrically connected to the electrical feedthrough 602, e.g., the electrode cup 606, by an interconnect lead, via, or cable (not shown) extending through the helix mount 502. The interconnect can be welded to the electrode cup 606 and the fixation element 106, and may be conductive to transfer a pacing impulse radially outward through the helix mount 502 to the fixation element 106. Accordingly, like the pacing element 108, the fixation element 106 may deliver pacing impulses to the target tissue, e.g., the right bundle branch 116.

It is contemplated that, although the fixation element 106 and the pacing element 108 may deliver a same, anodic, or cathodic, impulse to the target tissue, a polarity of the pacing may be switched or varied between the elements. For example, a coaxial feedthrough may be used to allow a first impulse to be delivered to the pacing element 108 and to allow a second impulse to be delivered to the fixation element 106. Polarities of the impulses may then be individually controlled to allow for any combination of anodic or cathodic pacing. For example, the left bundle branch 114 may be cathodically paced or anodically paced by the pacing element 108, and simultaneously, the right bundle branch 116 may be cathodically or anodically paced by the fixation element 106.

In an embodiment, the biostimulator 100, and more particularly the pacing element 108 is coupled to a therapeutic agent. For example, the filler 608 within the electrode cup 606 can contain a therapeutic agent, which may be eluted into a central lumen of the pacing element 108. The filler 608 can be referred to as a monolithic controlled release device (MCRD). The MCRD can include the therapeutic material, which may include a corticosteroid, such as dexamethasone sodium phosphate, dexamethasone acetate, etc. Furthermore, the therapeutic agent can be loaded in a silicone matrix. Accordingly, the filler 608 can deliver a specified dose of a therapeutic agent, e.g., a corticosteroid, into the target tissue. When the target tissue is drawn into the central lumen of the pacing element 108, the therapeutic agent can be effectively delivered into the tissue after the biostimulator is implanted in a patient. Accordingly, inflammation or injury of the captured tissue may be reduced.

Other modes of coupling the therapeutic agent to the pacing element 108 may be used. For example, the filler 608 may be a plug that is loaded directly into the central lumen of the pacing element 108. Furthermore, the filler 608 may be a coating that is applied directly to an outer surface of the helical or conical electrode described above. In an embodiment, the filler 608 is annularly shaped, and the annular filler is loaded over an external surface of the pacing element 108 as a sleeve.

The extendable support 504, e.g., the telescopic pedestal 506, can include one or more stages 508 telescopically movable relative to each other. For example, the one or more stages 508 can include a distal pedestal stage 622 that slides over or within a proximal pedestal stage 624. Relative movement of the stages 508, e.g., telescopic movement of the stages relative to each other, can cause a distal pedestal end 628 of the distal pedestal stage 622 to move longitudinally relative to a proximal pedestal end 626 of the proximal pedestal stage 624.

In an embodiment, the proximal pedestal end 626 is coupled to the housing 302. For example, the proximal pedestal stage 624 can be mounted on the insulator 610, e.g., by joining the proximal pedestal end 626 to a distal end of the insulator 610. The components may be joined by a brazed joint. The proximal pedestal stage 624 may be otherwise secured to the housing 302 or the header assembly 600 such that the proximal pedestal end 626 remains longitudinally fixed relative to the fixation element 106. More particularly, the proximal pedestal stage 624 and the fixation element 106 may be fixed relative to the housing 302 such that translation or rotation of the housing 302 causes corresponding translation or rotation of the fixation element 106 and the proximal pedestal stage 624.

The distal pedestal end 628 can be coupled to the pacing element 108. More particularly, whereas the proximal pedestal end 626 may be fixed relative to the fixation element 106, the distal pedestal end 628 can be fixed relative to the pacing element 108. The pacing element 108 can be secured to the distal pedestal end 628, e.g., by a weld joint, such that translation or rotation of the distal pedestal stage 622 causes corresponding translation or rotation of the pacing element 108.

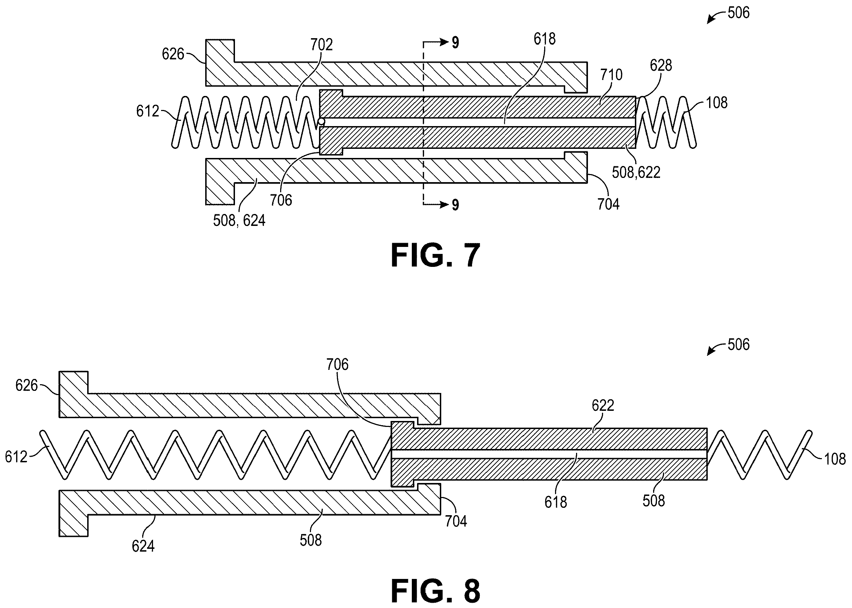

Referring to FIG. 7, a cross-sectional view of an extendable support of a biostimulator in an unextended state is shown in accordance with an embodiment. The one or more stages 508 of the telescopic pedestal 506 may be in sliding contact with each other. For example, the distal pedestal stage 622 can be slidably mounted on the proximal pedestal stage 624. The distal pedestal stage 622 can slide into, through, or within the proximal pedestal stage 624. The stages 508 may therefore move in a telescoping action to cause the pacing element 108 to move longitudinally relative to the proximal pedestal end 626.

The proximal pedestal stage 624 can have a tubular structure. The stage 508 may include an outer wall surrounding a stage channel 702. The stage channel 702 can be an elongated lumen sized and shaped to receive the distal pedestal stage 622. In an embodiment, the distal pedestal stage 622 is at least partly contained within the stage channel 702. For example, in the retracted state, the distal pedestal end 628 may be located adjacent to a distal stage end 704 of the proximal pedestal stage 624. More particularly, the distal pedestal end 628 may be nearer to the distal stage end 704 than a proximal stage end 706 of the distal pedestal stage 622.

When the telescoping pedestal is in the an extended state, the extendable conductor 612 may be compressed. The extendable conductor 612 can, for example, be a coil having turns that may stretch or compress longitudinally. For example, the extendable conductor 612 can have a wire structure similar to a an extension or compression spring. The extendable conductor 612 may, however, have a low spring constant such that the conductor can extend or compress under low loads. Accordingly, the extendable conductor 612 can allow the distal pedestal stage 622 to move freely within the proximal pedestal stage 624. The extendable conductor 612 may convey electrical impulses from the pacing circuitry to the conductive core 618 regardless of a relative location of the pedestal stages 508.

The conductive core 618 of the distal pedestal stage 622 may be a metallic core embedded within an outer jacket 710. The outer jacket 710 may, for example, include a polymeric coating or jacketing surrounding the conductive core 618. In an embodiment, the outer jacket 710 includes an insulative coating. For example, the insulative coating can be a parylene coating disposed on an outer surface of the conductive core 618. The insulative coating may therefore be located between the one or more stages 508 of the telescopic pedestal 506. More particularly, the outer jacket 710 may separate and insulate the conductive core 618 from the pedestal stage 508 within which the distal pedestal stage 622 slides.

Referring to FIG. 8, a cross-sectional view of an extendable support of a biostimulator in an extended state is shown in accordance with an embodiment. Telescopic end movement of the distal pedestal stage 622 relative to the proximal pedestal stage 624 can drive the proximal stage end 706 forward toward the distal stage end 704. In an embodiment, the distal pedestal stage 622 includes a proximal lip that is sized to interfere with a distal lip of the proximal pedestal stage 624. More particularly, the stage lips can contact each other when the distal pedestal stage 622 moves inside the proximal pedestal stage 624 to a fully extended state. The interference can retain the distal pedestal stage 622 and limit movement of the pacing element 108 in the forward direction.

In the extended state, the extendable conductor 612 can stretch. More particularly, when the distal pedestal stage 622 slides within the proximal pedestal stage 624 in the forward direction, the extendable conductor 612 may lengthen. The conductor may nonetheless be attached to the electrode cup 606 and the conductive core 618, and thus, pacing impulses may be transmitted through the extendable conductor 612 to the pacing element 108 when the telescopic pedestal 506 is in the extended state.

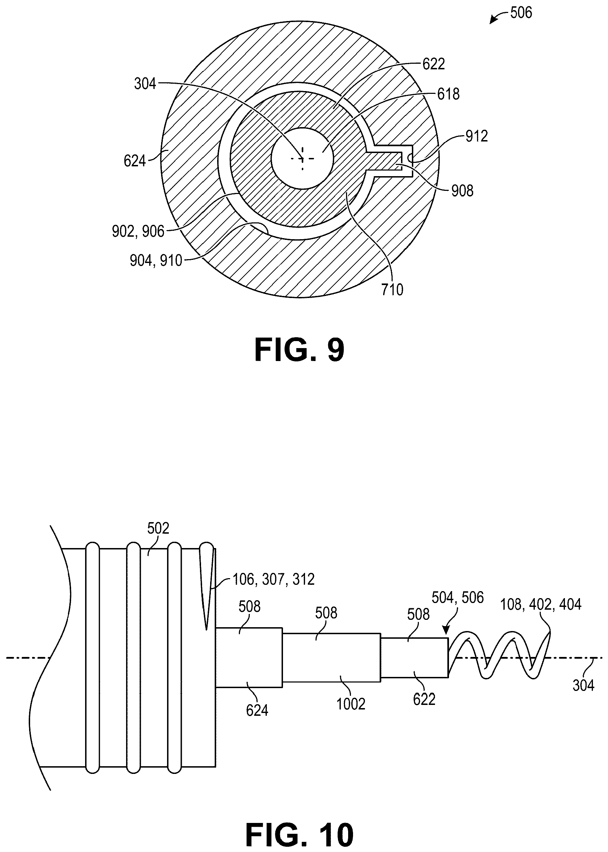

Referring to FIG. 9, a cross-sectional view, taken about line A-A of FIG. 7, of an extendable support of a biostimulator is shown in accordance with an embodiment. A comparison of the telescopic pedestal 506 in the unextended state and the extended state indicates that the stages 508 of the extendable support 504 can translate along the longitudinal axis 304 relative to each other. In an embodiment, rotational movement between the one or more stages 508 may, however, be restricted. More particularly, sliding surfaces of the stages 508, such as an outer sliding surface 902 of the distal pedestal stage 622 and an inner sliding surface 904 of the proximal pedestal stage 624, can have conforming, non-circular profiles that limit rotation of the stages relative to each other about the longitudinal axis 304.

The conforming, non-circular profiles of the pedestal stages 508 may form a keyed connection between the stage components. For example, the outer sliding surface 902 of the distal pedestal stage 622 can have an outer profile 906 that includes the shape of a key 908. The key 908 can include a ridge, pin, protrusion, etc. extending laterally, e.g., radially outward, from a circular portion of the outer profile 906. The inner sliding surface 904 of the proximal pedestal stage 624 may, by contrast, have an inner profile 910 that includes the shape of a keyway 912. More particularly, the keyway 912 of the proximal pedestal stage 624 can be a key slot within which the key 908 of the distal pedestal stage 622 slides. The key 908 can include an elongated ridge extending longitudinally along the distal pedestal stage 622. The ridge can conform to and fit within the elongated channel forming the keyway 912. The key 908 can slide within the keyway 912 in the longitudinal direction, however, rotation of the key 908 about the longitudinal axis 304 can be prevented by contact with the keyway 912. Accordingly, the distal pedestal stage 622 can translate within, but may not rotate relative to, the proximal pedestal stage 624.

It will be appreciated that the key 908 and keyway 912 illustrated in FIG. 9 are provided by way of example, and do not limit the non-circular shapes that may be used to restrict relative rotation of the pedestal stages. For example, the outer profile 906 and the inner profile 910 may have conforming elliptical or polygonal shapes that would similarly allow relative translation, and restrict relative rotation.

As described above, the outer jacket 710 of the distal pedestal stage 622 may be formed from an insulative material. The sliding surfaces of the pedestal stages 508 may also be formed from low-friction materials. For example, the outer sliding surface 902 of the distal pedestal stage 622 may be formed from parylene. The inner sliding surface 904 of the proximal pedestal stage 624 may also be formed from a polymer material. For example, the proximal pedestal stage 624, including the inner sliding surface 904, may be formed from PEEK. The pedestal stages 508 may therefore electrically insulate the conductive core 618 from surrounding tissue, and may have low surface friction to facilitate free movement of the telescoping stages 508 relative to each other.

Referring to FIG. 10, a side view of a distal portion of a biostimulator having a pacing element movable relative to a fixation element is shown in accordance with an embodiment. In an embodiment, the extendable support 504 includes the telescopic pedestal 506 having three or more stages 508. The stages 508 may include, for example, the distal pedestal stage 622 and the proximal pedestal stage 624. At least one additional stage, such as a medial pedestal stage 1002, may be integrated into the extendable structure. The medial pedestal stage 1002 can slide relative to the other stages 508. For example, the medial pedestal stage 1002 can slide within the proximal pedestal stage 624, and the distal pedestal stage 622 can slide within the medial pedestal stage 1002. Accordingly, the telescopic stages 508 can expand or collapse to change a distance between the fixation element 106 and the pacing element 108.

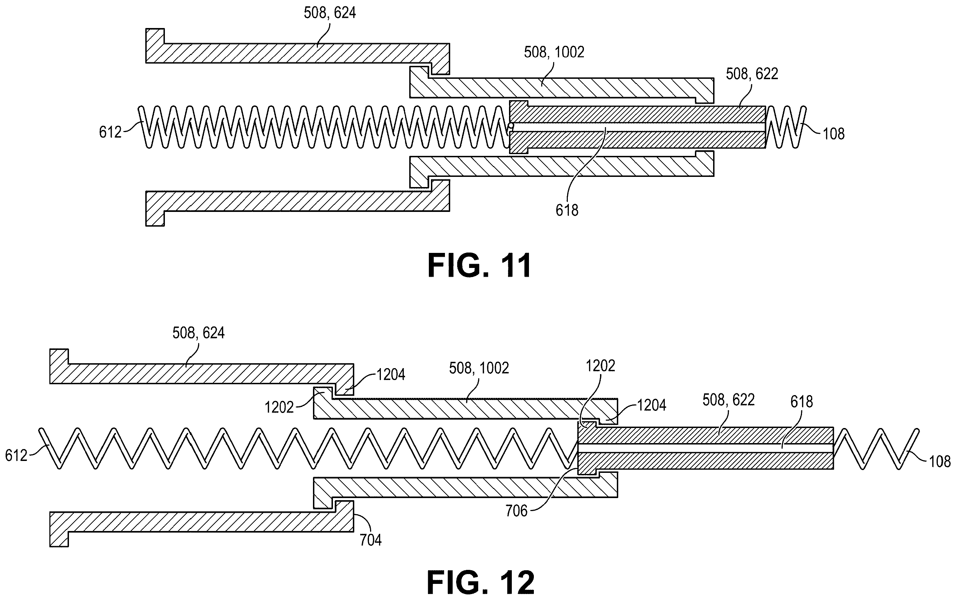

Referring to FIG. 11, a cross-sectional view of an extendable support of a biostimulator in an unextended state is shown in accordance with an embodiment. In the unextended state, at least one of the pedestal stages 508 may be retracted within an adjacent pedestal stage 508. For example, the distal pedestal stage 622 is shown retracted within the medial pedestal stage 1002. The medial pedestal stage 1002 is shown in an extended state within the proximal pedestal stage 624, however, it will be appreciated that the medial pedestal stage 1002 may also be retracted into the proximal pedestal stage 624. When all of the stages 508 are retracted into adjacent stages 508, the distance between the pacing element 108 and the fixation element 106 may be at a minimum.

Referring to FIG. 12, a cross-sectional view of an extendable support of a biostimulator in an extended state is shown in accordance with an embodiment. In the extended state, pedestal stages 508 may be extended within adjacent pedestal stages 508. For example, the distal pedestal stage 622 can be extended within the medial pedestal stage 1002, and the medial pedestal stage 1002 can be extended within the proximal pedestal stage 624. When all of the stages 508 are extended forward within adjacent stages 508, the distance between the elements may be at a maximum.

A structure and function of additional pedestal stages 508 may be similar to that described above. The medial pedestal stage 1002 can include stage lips 1202, at a proximal end/or distal end, which interfere with corresponding lips 1204 of adjacent stages 508. Accordingly, the lips 1202, 1204 of the medial pedestal stage 1002 and the distal pedestal stage 622 can interfere when the proximal stage end 706 is fully forward. Similarly, the lips 1202, 1204 of the medial pedestal stage 1002 and the proximal pedestal stage 624 can interfere when the medial pedestal stage 1002 is fully forward and the lips contact near the distal stage end 704. Translation of the stages 508 relative to each other may therefore be constrained by contact between the stage lips 1202, 1204.

Rotation of stages 508 relative to each other may also be constrained by corresponding conforming, non-circular profiles. More particularly, each of the three or more pedestal stages 508 can have sliding surfaces that are shaped, e.g., keyed, to constrain rotational movement of the stages 508 relative to each other about the longitudinal axis 304.

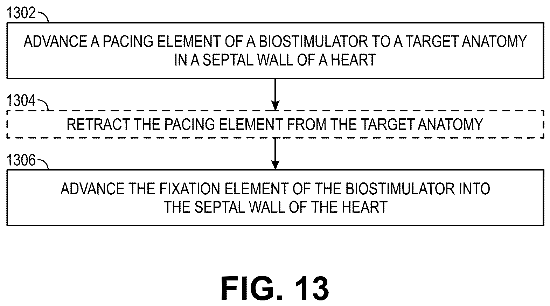

Referring to FIG. 13, a flowchart of a method of accessing a target anatomy using a biostimulator having a pacing element movable relative to a fixation element is shown in accordance with an embodiment. The biostimulator can be delivered through a vasculature of a patient toward the target anatomy. When the biostimulator is being tracked through the vasculature into a chamber of the heart 102, the expandable support may be in the unexpanded state. For example, stages 508 of the telescopic pedestal 506 can be retracted within each other. Accordingly, a longitudinal distance between the fixation element 106 and the pacing element 108 may be at a minimum during device delivery.

At operation 1302, the pacing element 108 is advanced to a target anatomy in a septal wall 110 of a heart 102. The distal helix 402 of the pacing element 108 can be urged against the septum 104. The housing 302 of the device may be turned in the first rotational direction 406, e.g., clockwise, to screw the distal helix 402 into the septal wall 110. More particularly, rotating the housing 302 in the first rotational direction 406 can drive the pacing element 108 through the septal wall 110. Rotation of the housing 302 may be provided by a rotational mechanism of the biostimulator transport system 202. The biostimulator transport system 202 can be used to transmit torque from the handle 204 to the attachment feature 310 to rotate the housing 302. When the housing 302 is turned, the distal helix 402 can be pulled forward into the septal tissue. As the helix screws into the tissue, the telescopic pedestal 506 can transform from the unextended state to the extended state. More particularly, the advancing pacing element 108 can pull the distal pedestal stage 622 forward within the proximal pedestal stage 624 and cause the telescopic pedestal 506 to expand. A longitudinal distance between the pacing element 108 and the fixation element 106 can increase as the distal helix 402 nears the left bundle branch 114. Care can be taken to avoid engaging the fixation element 106 to the septum 104 while the pacing element 108 is driven forward toward the left bundle branch 114. Accordingly, the proximal helix 307 of the fixation element 106 can remain at a same location within the heart 102 chamber while the pacing element 108 advances toward the target anatomy. Notably, longitudinal movement of the pacing element 108 can occur passively as a result of device rotation as compared to, for example, actively pushing forward on the distal pedestal stage 622.

At operation 1304, optionally, the pacing element 108 may be retracted from the target anatomy. Left bundle branch 114 pacing can be confirmed electro-physiologically by: a peak left ventricular activation time of < 80 milliseconds on a lead V5 and/or V6, a pacing impedance of approximately 600 ohms, and the presence of left bundle branch potential. Placement of the pacing element 108 may also be confirmed via imaging, noting that the left bundle branch 114 is typically 5 to 14 mm deep within the septal wall 110. When the pacing element 108 is in the left bundle branch 114, the housing 302 may be rotated in the second rotational direction 408, opposite to the first rotational direction 406, e.g., counterclockwise. The opposite rotation can retract the pacing element 108 from the target anatomy. For example, rotation of the housing 302 two turns in the counterclockwise direction can allow the distal helix 402 to move back by a predetermined distance.

At operation 1306, which may optionally be performed after retracting the pacing element 108, the fixation element 106 is advanced into the septal wall 110. The proximal helix 307 of the fixation element 106 can be urged against the septal wall 110, and the housing 302 can be rotated in the first rotational direction 406, e.g. clockwise. Rotation of the housing 302 when both the fixation element 106 and the pacing element 108 are engaged to the tissue can cause the helices to drive forward into the septal wall 110. The helices may have a same pitch and thus may advance longitudinally by a same distance per rotation of the housing 302. More particularly, the housing 302 can be rotated to cause the helices to advance by the predetermined distance. For example, if the pacing element 108 was retracted from the left bundle branch 114 by rotation of the housing 302 by two turns in the counterclockwise direction, housing 302 may be rotated two turns in the clockwise direction to advance the pacing element 108 to the left bundle branch 114. The rotation can also drive the fixation element 106 into the septal wall 110. Fixation element 106 may advance to the right bundle branch 116, for example. In any case, fixation element 106 can anchor the device within the septal wall 110 such that the pacing element 108 is secured at the left bundle branch 114.

The bundle branch(es) can be electrically stimulated through the pacing element 108 and/or fixation element 106. When the biostimulator elements are advanced into the target tissue, the fixation element 106 can anchor within the septal wall 110, e.g., at the right bundle branch 116, and the pacing element 108 can extend to the left bundle branch 114. Electrical impulses may then be delivered through one or both of the elements to pace the bundle branch(es). As described above, there is the option of using the fixation element 106 to perform anodic and/or cathodic pacing of the right bundle branch 116. Furthermore, the fixation element 106 may be used as an anode to provide bipolar pacing between the pacing element 108 acting as the cathode. If the output is increased, the fixation element 106 may pace the right bundle branch 116 and the pacing element 108 may pace the left bundle branch 114. Accordingly, the biostimulator can activate the left and/or right portions of the heart 102 synchronously to perform deep septal pacing.

## Claims

1. A biostimulator (100), comprising:
a housing (302) having a longitudinal axis (304) and containing pacing circuitry in an electronics compartment (306);
a fixation element (106) coupled to the housing (302);
an extendable support (504) comprising a telescopic pedestal (506) having a proximal pedestal end (626) coupled to the housing (302) and a distal pedestal end (628), wherein the telescopic pedestal (506) includes one or more stages (622, 624) telescopically movable relative to each other such that the distal pedestal end (628) is longitudinally movable relative to the proximal pedestal end (626); and
a pacing element (108) coupled to the distal pedestal end (628), wherein the pacing element (108) is longitudinally movable relative to the fixation element (106) and the housing (302).

2. The biostimulator of claim 1, wherein the fixation element (106) includes a proximal helix (307) extending about the longitudinal axis (304) to a first helix tip (312), and wherein the pacing element (108) includes a distal helix (402) extending about the longitudinal axis (304) to a second helix tip (404).

3. The biostimulator of claim 1 or 2, wherein the one or more stages (622, 624) include a distal pedestal stage (628) slidably mounted on a proximal pedestal stage (626).

4. The biostimulator of claim 3, wherein sliding surfaces (902, 904) of the distal pedestal stage (628) and the proximal pedestal stage (626) have conforming, non-circular profiles.

5. The biostimulator of claim 4, wherein the non-circular profiles include a key (908) and a keyway (912).

6. The biostimulator of any one of claims 1 to 5, further comprising an extendable conductor (612) electrically connecting the pacing circuitry to the pacing element (108) through the telescopic pedestal (506).

7. The biostimulator of any one of claims 1 to 6, further comprising an insulative coating between the one or more stages (622, 624).

8. **The** biostimulator of any one of claims 1 to 7, further comprising an attachment feature (310) mounted on a proximal housing end (313) of the housing (302), wherein the fixation element (106) and the pacing element (108) are coupled to a distal housing end (309) of the housing (302).

9. **The** biostimulator of any one of claims 1 to 8, wherein the pacing element (108) is electrically connected to the pacing circuitry.

10. **The** biostimulator of claim 9, wherein the fixation element (106) and the pacing element (108) are electrically connected to the pacing circuitry.

11. **The** biostimulator of claim 10, wherein the fixation element (106) is electrically connected to the pacing circuitry through an electrode cup (606).

12. **The** biostimulator of claim 11, wherein the electrode cup (606) is mounted in a helix mount (606), and wherein an interconnect lead extends from the electrode cup (606) to the fixation element (106) through the helix mount (502).

13. The biostimulator of any of claims 11 to 12, further comprising a monolithic controlled release device (MCRD) within the electrode cup (606), wherein the MCRD includes a therapeutic material.

14. A biostimulator system (200), comprising:
a biostimulator transport system (202); and
a biostimulator (100) of any one of claims 1 to 13 mounted on the biostimulator transport system (20

## Patentansprüche

1. Biostimulator (100), umfassend:
ein Gehäuse (302), das eine Längsachse (304) aufweist und eine Schrittmacherschaltung in einem Elektronikfach (306) enthält;
ein Fixierungselement (106), das an das Gehäuse (302) gekoppelt ist;
eine erweiterbare Stütze (504), umfassend einen Teleskopsockel (506), der ein proximales Sockelende (626), das an das Gehäuse (302) gekoppelt ist, und ein distales Sockelende (628) aufweist, wobei der Teleskopsockel (506) eine oder mehrere Stufen (622, 624) beinhaltet, die teleskopisch relativ zueinander bewegbar sind, sodass das distale Sockelende (628) relativ zu dem proximalen Sockelende (626) längs bewegbar ist; und
ein Schrittmacherelement (108), das an das distale Sockelende (628) gekoppelt ist, wobei das Schrittmacherelement (108) relativ zu dem Fixierungselement (106) und dem Gehäuse (302) längs bewegbar ist.

2. Biostimulator nach Anspruch 1, wobei das Fixierungselement (106) eine proximale Helix (307) beinhaltet, die sich um die Längsachse (304) zu einer ersten Helixspitze (312) erstreckt, und wobei das Schrittmacherelement (108) eine distale Helix (402) beinhaltet, die sich um die Längsachse (304) zu einer zweiten Helixspitze (404) erstreckt.

3. Biostimulator nach Anspruch 1 oder 2, wobei die eine oder mehreren Stufen (622, 624) eine distale Sockelstufe (628) beinhalten, die verschiebbar an einer proximalen Sockelstufe (626) montiert ist.

4. Biostimulator nach Anspruch 3, wobei Schiebeflächen (902, 904) der distalen Sockelstufe (628) und der proximalen Sockelstufe (626) übereinstimmende, nichtkreisförmige Profile aufweisen.

5. Biostimulator nach Anspruch 4, wobei die nichtkreisförmigen Profile einen Schlüssel (908) und eine Schlüsselöffnung (912) beinhalten.

6. Biostimulator nach einem der Ansprüche 1 bis 5, ferner umfassend einen erweiterbaren Leiter (612), der die Schrittmacherschaltung durch den Teleskopsockel (506) elektrisch mit dem Schrittmacherelement (108) verbindet.

7. Biostimulator nach einem der Ansprüche 1 bis 6, ferner umfassend eine isolierende Beschichtung zwischen der einen oder den mehreren Stufen (622, 624).

8. Biostimulator nach einem der Ansprüche 1 bis 7, ferner umfassend ein Anbringungsmerkmal (310), das an einem proximalen Gehäuseende (313) des Gehäuses (302) montiert ist, wobei das Fixierungselement (106) und das Schrittmacherelement (108) an ein distales Gehäuseende (309) des Gehäuses (302) gekoppelt sind.

9. Biostimulator nach einem der Ansprüche 1 bis 8, wobei das Schrittmacherelement (108) elektrisch mit der Schrittmacherschaltung verbunden ist.

10. Biostimulator nach Anspruch 9, wobei das Fixierungselement (106) und das Schrittmacherelement (108) elektrisch mit der Schrittmacherschaltung verbunden sind.

11. Biostimulator nach Anspruch 10, wobei das Fixierungselement (106) durch einen Elektrodenbecher (606) elektrisch mit der Schrittmacherschaltung verbunden ist.

12. Biostimulator nach Anspruch 11, wobei der Elektrodenbecher (606) in einer Helixhalterung (606) montiert ist und wobei sich eine Verbindungsleitung von dem Elektrodenbecher (606) zu dem Fixierungselement (106) durch die Helixhalterung (502) erstreckt.

13. Biostimulator nach einem der Ansprüche 11 bis 12, ferner umfassend eine monolithische Vorrichtung mit kontrollierter Freisetzung (MCRD) innerhalb des Elektrodenbechers (606), wobei die MCRD ein therapeutisches Material beinhaltet.

14. Biostimulatorsystem (200), umfassend:
ein Biostimulatortransportsystem (202); und
einen Biostimulator (100) nach einem der Ansprüche 1 bis 13, der an dem Biostimulatortransportsystem (20 montiert ist

## Revendications

1. Biostimulateur (100), comprenant :
un boîtier (302) ayant un axe longitudinal (304) et contenant un circuit de stimulation dans un compartiment électronique (306) ;
un élément de fixation (106) couplé au boîtier (302) ;
un support extensible (504) comprenant un piédestal télescopique (506) ayant une extrémité de piédestal proximale (626) couplée au boîtier (302) et une extrémité de piédestal distale (628), dans lequel le piédestal télescopique (506) inclut un ou plusieurs étages (622, 624) mobiles de manière télescopique les uns par rapport aux autres de sorte que l'extrémité de piédestal distale (628) soit mobile longitudinalement par rapport à l'extrémité de piédestal proximale (626) ; et
un élément de stimulation (108) couplé à l'extrémité de piédestal distale (628), dans lequel l'élément de stimulation (108) est mobile longitudinalement par rapport à l'élément de fixation (106) et au boîtier (302).

2. Biostimulateur selon la revendication 1, dans lequel l'élément de fixation (106) inclut une hélice proximale (307) s'étendant autour de l'axe longitudinal (304) jusqu'à une première pointe d'hélice (312), et dans lequel l'élément de stimulation (108) inclut une hélice distale (402) s'étendant autour de l'axe longitudinal (304) jusqu'à une seconde pointe d'hélice (404).

3. Biostimulateur selon la revendication 1 ou 2, dans lequel les un ou plusieurs étages (622, 624) incluent un étage de piédestal distal (628) monté de manière coulissante sur un étage de piédestal proximal (626).

4. Biostimulateur selon la revendication 3, dans lequel des surfaces de coulissement (902, 904) de l'étage de piédestal distal (628) et de l'étage de piédestal proximal (626) ont des profils non circulaires complémentaires.

5. Biostimulateur selon la revendication 4, dans lequel les profils non circulaires incluent une clavette (908) et une rainure de clavette (912).

6. Biostimulateur selon l'une quelconque des revendications 1 à 5, comprenant en outre un conducteur extensible (612) connectant électriquement le circuit de stimulation à l'élément de stimulation (108) à travers le piédestal télescopique (506).

7. Biostimulateur selon l'une quelconque des revendications 1 à 6, comprenant en outre un revêtement isolant entre les un ou plusieurs étages (622, 624).

8. Biostimulateur selon l'une quelconque des revendications 1 à 7, comprenant en outre un accessoire de fixation (310) monté sur une extrémité de boîtier proximale (313) du boîtier (302), dans lequel l'élément de fixation (106) et l'élément de stimulation (108) sont couplés à une extrémité de boîtier distale (309) du boîtier (302).

9. Biostimulateur selon l'une quelconque des revendications 1 à 8, dans lequel l'élément de stimulation (108) est connecté électriquement au circuit de stimulation.

10. Biostimulateur selon la revendication 9, dans lequel l'élément de fixation (106) et l'élément de stimulation (108) sont connectés électriquement au circuit de stimulation.

11. Biostimulateur selon la revendication 10, dans lequel l'élément de fixation (106) est connecté électriquement au circuit de stimulation à travers une coupelle d'électrode (606).

12. Biostimulateur selon la revendication 11, dans lequel la coupelle d'électrode (606) est montée dans un support d'hélice (606), et dans lequel un conducteur d'interconnexion s'étend à partir de la coupelle d'électrode (606) jusqu'à l'élément de fixation (106) à travers le support d'hélice (502).

13. Biostimulateur selon l'une quelconque des revendications 11 à 12, comprenant en outre un dispositif de libération contrôlée monolithique (DLCM) à l'intérieur de la coupelle d'électrode (606), dans lequel le DLCM inclut un matériau thérapeutique.

14. Système de biostimulateur (200), comprenant :
un système de transport de biostimulateur (202) ; et
un biostimulateur (100) selon l'une quelconque des revendications 1 à 13 monté sur le système de transport de biostimulateur (202).
